**Europäisches Patentamt**

**European Patent Office**

**Office européen des brevets**

(11) Veröffentlichungsnummer: **0 093 375**
**B1**

(12) # EUROPÄISCHE PATENTSCHRIFT

(45) Veröffentlichungstag der Patentschrift:
**14.05.86**

(51) Int. Cl.⁴: **C 12 N 5/00**, C 12 N 7/00

(21) Anmeldenummer: **83104059.7**

(22) Anmeldetag: **26.04.83**

(54) **Kulturmedium und Verfahren zur Virusvermehrung in Suspensionskultur.**

(30) Priorität: **30.04.82 DE 3216163**

(43) Veröffentlichungstag der Anmeldung:
**09.11.83 Patentblatt 83/45**

(45) Bekanntmachung des Hinweises auf die Patenterteilung:
**14.05.86 Patentblatt 86/20**

(84) Benannte Vertragsstaaten:
**BE DE FR NL**

(56) Entgegenhaltungen:
**DE - A - 2 553 827**
**GB - A - 1 480 690**
**US - A - 4 205 126**
**US - E - 30 753**

**ANALYTICAL BIOCHEMISTRY, Band 102, Nr. 2; 1980, Seite 255-270, Academic Press, Inc., USA D. BARNES et al.: "Review. Methods for Growth of cultured cells in serum-free medium"**
**BIOLOGICAL ABSTRACTS, Band 71, 1981, Ref. Nr. 55810, Biological Abstracts. Inc., Philadelphia, PA., USA M. TAUB et al.: "Growth of functional primary cultures of**

(73) Patentinhaber: **BEHRINGWERKE Aktiengesellschaft, Postfach 1140, D-3550 Marburg 1 (DE)**

(72) Erfinder: **Bernhardt, Dieter, Dr., Goldbergstrasse 18A, D-3553 Gölbe (DE)**
Erfinder: **Behrens, Fritz, Dr., Elsenhöhe 18, D-3550 Marburg (DE)**
Erfinder: **Bleser, Klaus, Kreppelweg 5, D-3570 Stadt-Allendorf (DE)**
Erfinder: **Krajczewski, Bruno, Eichenhain 1, D-3570 Stadt-Allendorf (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

(56) Entgegenhaltungen: (Fortsetzung)
**kidney epithelial cells in defined medium"**
**CHEMICAL ABSTRACTS, Band 92, Nr. 15, 14. April 1980, Spalte 122324, Nr. 122325e, Columbus, Ohio, USA M. TAUB et al.: "Growth of kidney epithelial cells in hormone-supplemented, serum-free medium"**

**Die Akte enthält technische Angaben, die nach dem Eingang der Anmeldung eingereicht wurden und die nicht in dieser Patentschrift enthalten sind.**

## Beschreibung

Die Erfindung betrifft ein Kulturmedium und ein Verfahren zur Vermehrung von Viren in einer Suspension von Zellen. Die vermehrten Viren dienen der Gewinnung von Antigenen, die in Diagnostika und gegebenenfalls in Impfstoffen verwendet werden können.

Die einzelnen Komponenten dieses Kulturmediums wurden bereits in anderen Kombinationen zuvor in Kulturmedien verwendet [vgl. u.a. Analytical Biochemistry, Vol. 1.2, Nr. 2 (1980), S. 255-270, US-A-4 205 126 und DE-A-2 553 827].

Viren können oft nur in Einschicht («Monolayer»)-Zellkulturen vermehrt werden, d.h., die Zellen und Viren vermehren sich nur an der Grenzfläche des Nährmediums. Dies bedeutet niedrige Raum-Zeit-Ausbeuten, grossen Arbeitsaufwand, hohe Mediumskosten sowie erhöhtes Kontaminationsrisiko, da in vielen Gefässen gearbeitet werden muss. Das Auffinden von Bedingungen unter denen ein Virus in einer Suspension von Zellen vermehrt wird, wobei der gesamte mit Nährlösung gefüllte Raum genutzt wird, bedeutet einen erheblichen Vorteil gegenüber einer Grenzflächenkultur.

Monolayer-Zellkuren sind für viele Viren bekannt. Für das Rinder-Leukose-Virus (BLV = bovines Leukosevirus) wurde ein solches Verfahren in J. Nat. Cancer Inst., Band 52, 491-497 (1974) beschrieben.

Dieses Virus verursacht die enzootische Leukose, die eine Erkrankung des hämatopoetischen, lymphatischen Systems in Rindern ist und erhebliche Verluste mit sich bringt. Zur Erkennung der infizierten Tiere kann man ein in Gewebekultur hergestelltes Antigen verwenden.

Es wurde überraschend ein Medium gefunden, in welchem Zellen und Viren in Suspension vermehrt werden können.

Gegenstand der Erfindung ist deshalb ein Nährmedium und seine Verwendung in einem Verfahren zur Vermehrung von Viren in einer Suspension von Zellen.

Das Medium enthält anorganische Salze, Aminosäuren, Zucker, Antibiotika und Vitamine sowie einen oder mehrere der Stoffe Insulin in einer Konzentration von mindestens 1,0 mg/l, Transferrin in einer Konzentration von mindestens 2,0 mg/l, Biotin in einer Konzentration von mindestens 0.5 mg/l, peptisches Pepton in einer Konzentration von mindestens 0.5 g/l und/oder Casein-Pepton in einer Konzentration von mindestens 0.5 g/l.

Gegebenenfalls sind diese Stoffe in folgenden Mengen im Medium enthalten:

| Insulin | 1,0 - 10,0 | mg/l |
|---|---|---|
| Transferrin | 2,0 - 20,0 | mg/l |
| Biotin | 0,5 - 10,0 | mg/l |
| peptisches Pepton | 0,5 - 10,0 | g/l |
| Casein-Pepton | 0,5 - 10,0 | g/l. |

Gegebenenfalls werden dem Medium 0 bis 5 Volumenprozent Serum zugegeben. Für die Zellkulturen geeignete Zellen sind besonders foetale Lämmer-Nieren-Zellen (FLK), die permanente humane Zellinie Hep-2 oder Baby-Hamster-Kidney-Zellen (BHK-Zellen, Monolayertyp).

Die Zellkulturen können nach bekannten Verfahren in Suspension angelegt werden.

In einer solchen Suspensionskultur können Reoviren, MKS-Viren, die Erreger der infektiösen bovinen Rhinotracheitis (IBR) oder der infektiösen pustulösen Vulvovaginitis oder Herpes simplex-Viren, besonders jedoch das Rinder-Leukose-Virus (BLV), das Para-Influenza 3-Virus oder Herpesvirus suis, vermehrt werden.

Die Viren können in bekannter Weise aufgearbeitet werden, um für diagnostische oder Impfzwecke geeignete Antigene zu erhalten.

Die folgenden Beispiele erläutern die Erfindung.

*Beispiel 1*

Aus einem 20-Liter-Fermenter der Zellenstammhaltung mit FLK-Zellen, die mit BLV infiziert sind, wurden 6 Liter Zellsuspension entnommen, in einen 50-Liter-Fermenter überführt und mit 19 Liter neuem KPL-2-Medium + 5% Kälberserum (KS) verdünnt.

*Zusammensetzung des KBL-2-Mediums*

| Grundmedium: | Na Cl | 7,2 | g/l |
|---|---|---|---|
| | K Cl | 0,4 | g/l |
| | Mg SO$_4$ × 7H$_2$O | 0,2 | g/l |
| | Fe(NO$_3$)$_3$ × 9H$_2$O | 0,0001 | g/l |
| | Na H$_2$ PO$_4$ × 2H$_2$O | 0,07 | g/l |
| | K H$_2$ PO$_4$ | 0,03 | g/l |
| | Na$_2$ H PO$_4$ × 2H$_2$O | 0,037 | g/l |
| | Ca Cl$_2$ × 2H$_2$O | 0,225 | g/l |
| | Neomycin (reine Base) | 0,1 | g/l |
| | Phenolrot | 0,02 | g/l |
| | Glucose | 3,5 | g/l |
| | Na HCO$_3$ | 1,55 | g/l |
| *Vitamine:* | 100fach nach Eagles 1959 doppelt | | |
| *Aminosäuren:* | L-Cystin | 1,6 | g/l |
| | L-Tryptophan | 0,8 | g/l |
| | L-Glutamin | 30,0 | g/l |
| *Zusätze* | Insulin | 2,5 | mg/l |
| | Transferrin | 5,0 | mg/l |
| | Biotin | 2,0 | mg/l |
| | peptisches Pepton (Oxoid,L49) | 1,25 | g/l |
| | Casein-Pepton (Serva,C/TLG). | 1,25 | g/l |

Die Suspensionskultur wurde unter Rühren bei 37°C bebrütet. Von der vierundzwanzigsten Stunde nach Ansatz ab wurde die Kultur mit 1 Liter Luft/Liter Kultur/Stunde durchgast. 5 Tage nach Kulturansatz wurde die Kultur geerntet, die Zellen abzentrifugiert und der Überstand in bekannter Weise zum BLV-Antigen aufgearbeitet [van Maaten und Miller, Bibl. Haematol. (1976) 43, 360-362].

Aus diesen 25 Liter Kulturflüssigkeit wurden durch Konzentrieren 250 ml einer Lösung von Leukose-Antigen gewonnen, die in der Agargelpräzipitation (AGP) einen Titer von 1 : 32 aufwies.

*Beispiel 2*

Aus einem 20-Liter-Fermenter mit FLK-Zellen, die mit BLV infiziert waren, wurden 5 Liter Zellsuspension entnommen, in einen 50-Liter-Fermenter überführt und mit 30 Liter neuem KPL-2-Medium + 2,5% KS verdünnt. Die Suspensionskultur wurde unter Rühren bei 36°C bebrütet. 30 Stunden nach Ansatz wurde die Kultur mit 0,5 Liter Luft/Liter Kultur/Stunde durchgast. 5 Tage nach Kulturansatz wurde die Kultur geerntet, die Zellen abzentrifugiert und der Überstand in bekannter Weise zum BLV-Antigen aufgearbeitet.

Aus den 35 Litern Kulturüberstand wurden durch Konzentrieren 350 ml einer Lösung von Leukose-Antigen gewonnen, die in der AGP einen Titer von 1 : 32 aufwies.

*Beispiel 3*

Von einer Rouxschale wurden HEP-2-Zellen in bekannter Weise abtrypsiniert und in 1 Liter KPL-2-Medium aufgenommen, in einen 2-Liter-Fermenter überführt und bei 37°C in Suspension gehalten. Nach 4 Tagen hatten sich die Zellen von 200 000 Zellen/ml auf 2 000 000 Zellen/ml vermehrt. Diese Zellsuspension wurde nun 1 : 3 mit neuem KPL-2-Medium verdünnt und mit Para-Influenza 3-Virus infiziert. Nach 48 Stunden wurde die Kultur geerntet. Der KBR-Titer der Virusernte betrug 1 : 16.

*Beispiel 4*

Von einer Rouxschale wurden HEP-2-Zellen in bekannnter Weise abtrypsiniert und in 1 Liter RPMI 1640-Medium, das 2,5 mg Insulin, 5 mg Transferrin und 2 mg Biotin enthielt, aufgenommen, in einen 2-Liter-Fermenter überführt und bei 37°C in Suspension gehalten.

Nach 4 Tagen hatten sich die Zellen von 170 000 Zellen/ml auf 2 000 000 Zellen/ml vermehrt. Diese Zellsuspension wurde nun 1 : 3 mit neuem RPMI 1640 + Insulin, Transferrin und Biotin verdünnt und mit Pl$_3$-Virus infiziert. Nach 48 Stunden wurde die Kultur geerntet. Der KBR-Titer betrug 1 : 20.

*Beispiel 5*

1 Liter Zellsuspension HEP-2-Zellen der 5. Suspensionspassage mit 2 200 000 Zellen/ml wurden mit 9 Liter RPMI 1640 + 2,5 mg Insulin, 5 mg Transferrin und 2 mg Biotin verdünnt und in einen 20-Liter-Fermenter überführt. Die Kultur wurde bei 37°C in Suspension gehalten. Nach 4 Tagen hatten sich die Zellen erneut auf 2 000 000 Zellen/ml vermehrt. Die Zellen wurden 1 : 3 mit frischem RPMI 1640-Medium + Insulin, Transferrin und Biotin verdünnt und mit Pl$_3$-Virus infiziert. 48 Stunden nach der Infektion erfolgte die Virusernte, die einen KBR-Titer von 1 : 20 aufwies.

*Beispiel 6*

Von einer Rouxschale wurden BHK-Monolayerzellen abtrypsiniert, in 1 Liter KPL-2-Medium aufgenommen, in einen 2-Liter-Fermenter überführt und bei 37°C in Suspension gehalten. Nach 48 Stunden hatten sich die Zellen von 180 000 Zellen/ml auf 2 400 000 Zellen/ml vermehrt. Die Kultur wurde 1 : 3 mit frischem KPL-2-Medium verdünnt und mit Herpesvirus suis infiziert. 24 Stunden später wurde wurde die Kultur geerntet. Im Festphasen-Immunadsorbens-Test wurde ein Titer von 1 : 3 gefunden.

**Patentansprüche**

1. Kulturmedium bestehend aus anorganischen Salzen, Aminosäuren, Zucker, Antibiotika, Vitaminen und Zusätzen, dadurch gekennzeichnet, dass unter diesen Zusätzen einer oder mehrere der Stoffe Insulin in einer Konzentration von mindestens 1,0 mg/l, Transferrin in einer Konzentration von mindestens 2,0 mg/l, Biotin in einer Konzentration von mindestens 0,5 mg/l, peptisches Pepton in einer Konzentration von mindestens 0,5 g/l und/oder Casein-Pepton in einer Konzentration von mindestens 0,5 g/l ist bzw.sind.

2. Kulturmedium nach Anspruch 1, dadurch gekennzeichnet, dass es 1 bis 10 mg/l Insulin, 2 bis 20 mg/l Transferrin, 0,5 bis 10 mg/l Biotin, 0,5 bis 10 g/l peptisches Pepton und/oder 0,5 bis 10 g/l Casein-Pepton enthält.

3. Verfahren zur Vermehrung von Viren in einer Suspension von Zellen, dadurch gekennzeichnet, dass in einem Medium nach einem der Ansprüche 1 oder 2 vermehrt wird.

4. Diagnostikum, das nach Anspruch 3 vermehrte Viren enthält.

5. Impfstoff, der nach Anspruch 3 vermehrte Viren enthält.

**Claims**

1. Culture medium consisting substantially of inorganic salts, amino acids, sugar, antibiotics, vitamins and additives, wherein among these additives there is or are one or more substances selected from the group of insulin in a concentration of at least 1 mg/l, transferrin in a concentration of at least 2 mg/l, biotin in a concentration of at least 0,5 mg/l, peptic peptone in a concentration of at least 0,5 g/l and/or casein peptone in a concentration of at least 0.5 g/l.

2. Culture medium as claimed in claim 1, which comprises from 1 to 10 mg/l of insulin, from 2 to 20 mg/l of transferrin, from 0.5 to 10 mg/l of biotin, from 0.5 to 10 g/l of peptic peptone and/or 0.5 to 10 g/l of casein peptone.

3. A process for the reproduction of viruses in a suspension of cells, which comprises reproducing the viruses in a medium as claimed in one of claims 1 or 2.

4. Diagnostic agent containing viruses reproduced as claimed in claim 3.

5. Vaccine containing viruses reproduced as claimed in claim 3.

**Revendications**

1. Milieu de culture constitué de sels minéraux, d'aminoacides, de sucre, d'antibiotiques, de vitamines et d'additifs, caractérisé en ce que parmie ces additifs, se trouvent une ou plusieurs des substances: insulin (à une concentration d'au moins 1,0 mg/l), transferrine (à une concentration d'au moins 2,0 mg/l), biotine (à une concentration d'au moins 0,5 mg/l), peptone peptique (à une concentration d'au moins 0,5 g/l) et/ou peptone de caséine (à une concentration d'au moins 0,5 g/l), respectivement.

2. Milieu de culture selon la revendication 1, caractérisé en ce qu'il contient 1 à 10 mg/l d'insuline, 2 à 20 mg/l de transferrine, 0,5 à 10 mg/l de biotine, 0,5 à 10 g/l de peptone peptique et/ou 0,5 à 10 g/l de peptone de caséine.

3. Procédé de multiplication de virus dans une suspension de cellules, caractérisé en ce que la multiplication se fait dans un milieu selon la revendication 1 ou 2.

4. Moyen de diagnostic contenant des virus multipliés selon la revendication 3.

5. Vaccin contenant des virus multipliés selon la revendication 3.